# EUROPEAN PATENT SPECIFICATION

(11) **EP 1 991 132 B1**
(45) Date of publication and mention of the grant of the patent: **10.10.2018**
(21) Application number: 07757250.1
(22) Date of filing: 21.02.2007
(51) Int. Cl.: A61B 90/00

(54) **ENDOSCOPIC APPLICATOR**
ENDOSKOPISCHER APPLIKATOR
APPLICATEUR ENDOSCOPIQUE

(30) Priority: 09.03.2006 US 371835
(43) Date of publication of application: 19.11.2008
(73) Proprietor: Ethicon, Inc, Somerville, NJ 08876-0151 (US)
(72) Inventor: GABEL, Jonathan, B., Randolph, New Jersey 07869 (US)
(74) Representative: Kirsch, Susan Edith
(86) International application number: PCT/US2007/062470
(87) International publication number: WO 2007/103630

(56) References cited:
- WO-A-02/35986
- WO-A-99/56692
- US-A- 5 263 933
- US-A- 6 086 607
- US-A1- 2002 120 240
- US-A1- 2005 154 353

## Description

### BACKGROUND OF THE INVENTION

### 1. Field of the Invention

The present invention relates generally to surgical devices, and more particularly to a packaged endoscopic applicator particularly suitable for delivering surgical fluids, hemostatic agents or the like.

### 2. Background Discussion

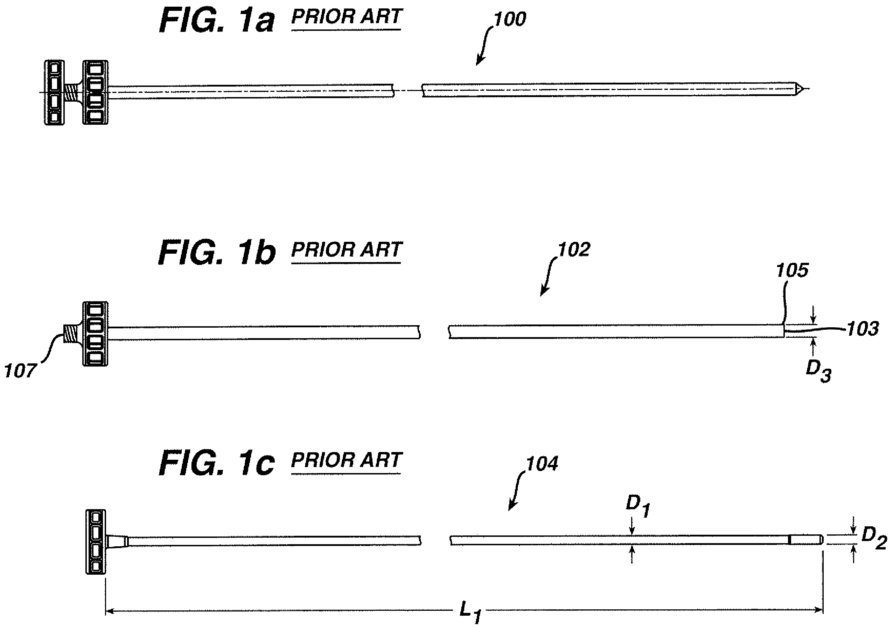

Minimally invasive surgical procedures, such as endoscopic procedures, have become very common. These procedures typically involve one or more small incisions that provide access to the relevant internal surgical site. A trocar, cannula or the like is placed into each incision, and all surgical steps are subsequently performed through the trocar(s) using appropriately sized and designed instruments and devices.
Often times during such surgical procedures it is necessary to deliver medicinal materials, such as liquids, gels or the like, to the surgical site. Delivery to a specific location, and delivery of a specific amount of the material is often desired, such as is the case with anti-adhesion or hemostatic materials. For such materials, general washing or irrigation type delivery methods are unsuitable. Further, many such materials are costly, rendering precise application even more important.
In efforts to address these concerns, many endoscopic applicators have been developed, and those known to the inventors typically involve a long catheter-like outer tube (long enough to pass through the endoscopic trocar or port) having a channel extending therethrough between its proximal and distal ends. The surgical material, typically a fluid or gel-like material, is expressed into the channel (by any suitable means such as a syringe) for delivery to the surgical site. The proximal end includes some type of handle device to enable the user to adequately grip and manipulate the device. A second plunger-like element is sized to fit into the outer tube channel, and when slid therein expresses the material within the outer tube out through its distal end. A representative example of such a prior art applicator 100 is
illustrated in Figs. 1a-1c. The applicator 100 includes an outer delivery tube 102 having a channel 103 extending therethrough between its distal 105 and proximal 107 ends, and an inner plunger-like device 104. The inner plunger device has a substantially uniform outer diameter D₁ along most of its length, but may step up to a larger diameter D₂ at its distal end. It is sized to fit within the substantially uniform inner diameter D₃ of the channel, and a has length L₁ long enough to enable it to pass at least through to the distal end of the channel of the outer delivery tube. The relative dimensions of D₂ and D₃ allow for movement of the two pieces relative to one another, but substantially prevent the material that is being expressed from the applicator 100 to seep in between. As indicated above, during operation the desired surgical material is injected into the channel of the outer tube. The inner plunger device is subsequently inserted into the channel, and slid into the channel to thereby force the material therein to be expressed out of the distal end of the channel and into the desired location at the surgical site. Ideally, the exact amount of material injected into the channel will be dispensed to the proper location.
WO 99/56692 describes a system for facilitating hemostasis of a puncture site in a blood vessel wherein an absorbable sponge pledger in a hydrated state is injected at an exterior of the blood vessel puncture to facilitate hemostasis. The system includes a tract dilator for locating the puncture, an introducer, and a plunger.
US 2002/0120240 A1 concerns a system and kit for delivery of restorative materials comprising cannulae for accessing an intraosseous space, and mandarins insertable into the cannulae and movable therein.
US 6,086,607 concerns a system including an adaptor and a syringe used for facilitating hemostasis of a biopsy tract or other puncture wound by delivery of an absorbable sponge in a hydrated state into the wound.

A further syringe is shown in US-A-5263933. Some known prior art devices of the type shown in Fig. 1a-1c are packaged both pre-sterilized and pre-assembled as shown in Fig. 1a. One problem that has been encountered is that the plunger device will separate and fall out of the outer delivery tube if it is not removed from the package in precisely the right manner. One alternative would be to provide the two components in separate package compartments, which is how the disposable FloSeal Endoscopic Applicator manufactured by Baxter International, Inc. is provided. This solution, however, is undesirable in that it requires much larger packaging, which costs more to make, more to dispose of, and takes up more storage space in the hospital or the like. Another solution is to provide a non-sterilized (typically reusable) product. Although this eliminates the concern about the components accidentally falling out in the sterile field, it is disadvantageous in that the product is not immediately ready to use as shipped, but rather must be cleaned and sterilized before use.

One other known applicator is somewhat similar to that described above, but further includes toward its proximal end (towards the handle portion) an airtight membrane or seal between the outer deliver tube and the inner plunger element to prevent gases within the surgical site from escaping. This device is known as the EndoAvitine® Endscopic Delivery System, which is sold by C.R. Bard, Inc. of New Jersey, and is designed specifically to delivery a collagen hemostat, which is a solid- like material that is pre-loaded into the applicator by the manufacturer. With this device, sliding movement between the outer delivery tube and inner plunger element is possible, but only by overcoming a much larger frictional force caused by the presence of the seal. This frictional force is typically not an issue for the specified application, since, due to its pre-loaded nature, the plunger is only used to express the solid-like material and therefore only has to travel a small distance under compressive force by the user. This type of design, however, would be unsuitable for endoscopic fluid applicators, where the plunger-like element is first entirely removed from the outer delivery tube so that the surgical fluid can be inserted therein, and subsequently reinserted and moved substantially entirely through the outer delivery tube to express the fluid. The frictional forces generated by having such a seal at a proximal end would render the device too difficult to manipulate.

Accordingly, it would be desirable to provide a new and improved endoscopic applicator that overcomes the problems described above.

### SUMMARY OF THE INVENTION

A packaged endoscopic fluid applicator is provided according to claim 1.

According to one embodiment, the elongate plunger element has a proximal end region, a middle region, and a distal end region, and the outer diameter is greater in the proximal end region than in the middle region. The outer delivery device channel may be substantially uniform along its length. In yet another embodiment, the outer diameter of the elongate plunger element at the middle region is approximately 3.2 mm, and the outer diameter at the proximal end region reaches approximately 4.3 mm. The outer diameter of the distal end region of the elongate plunger element may further be approximately 3.7 mm.

In yet another embodiment, the applicator further includes at least one interference element forming the interference fit as between the outer delivery device and the inner plunger device when the elongate plunger element is substantially fully received within the outer delivery device channel. In an alternate embodiment the outer diameter of the elongate tubular element may be less than approximately 5 mm.

According to alternate embodiments, the means for forming an interference fit may be an outwardly tapered proximal end region of the elongate plunger element, which may be a luer taper, may further include at least one interference element positioned between the proximal end regions of the outer delivery device channel and the elongate plunger element, or may be a recess within the handle of the inner plunger device for receiving a corresponding extension portion of the handle of the outer delivery device.

According to various other embodiments, the outer diameter of the elongate tubular element may be less than approximately 5 mm and/or the inner diameter of the outer delivery device channel may be approximately 3.8 mm.

The means for forming an threaded fit further comprises male and female thread forms at the proximal end region of the inner plunger device and outer delivery device channel.

According to one embodiment, the male and female thread forms are Luer taper threads. In another embodiment, the outer diameter of the elongate tubular element is less than 5 mm, and in yet another embodiment, the inner diameter of the outer delivery device channel is approximately 3.8 mm.

### BRIEF DESCRIPTION OF THE DRAWINGS

Figs. 1a-1c are side-views illustrating an exemplary prior art design and various components thereof;
Figs. 2a and 2b are side-views illustrating components of one embodiment of an applicator according to the present invention;
Fig. 3 is a cross sectional view of the components of Figs. 2a and 2b when assembled together;
Fig. 4 is a side view of the components of Figs. 2a and 2b when assembled together; and
Figs. 5a-5e illustrate alternate means by which to form an interference fit as between the outer delivery device and inner plunger device.

### DETAILED DESCRIPTION OF THE INVENTION

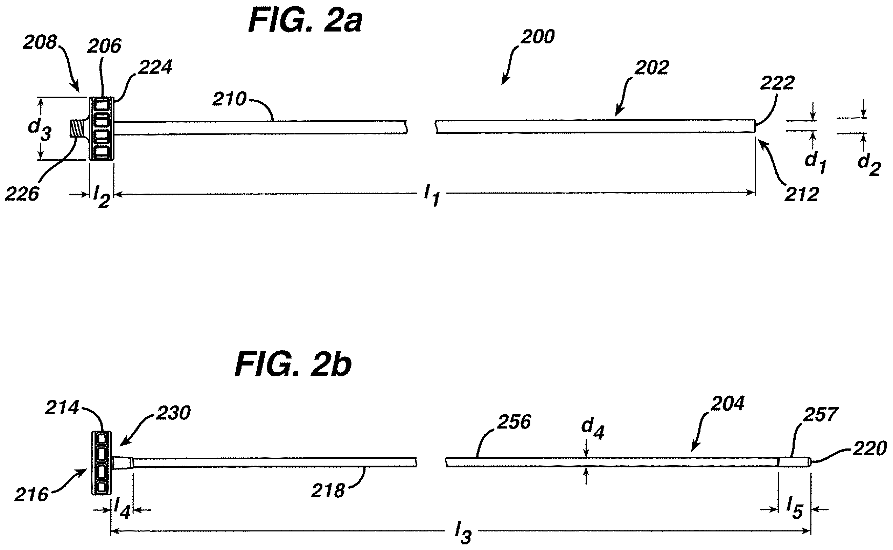

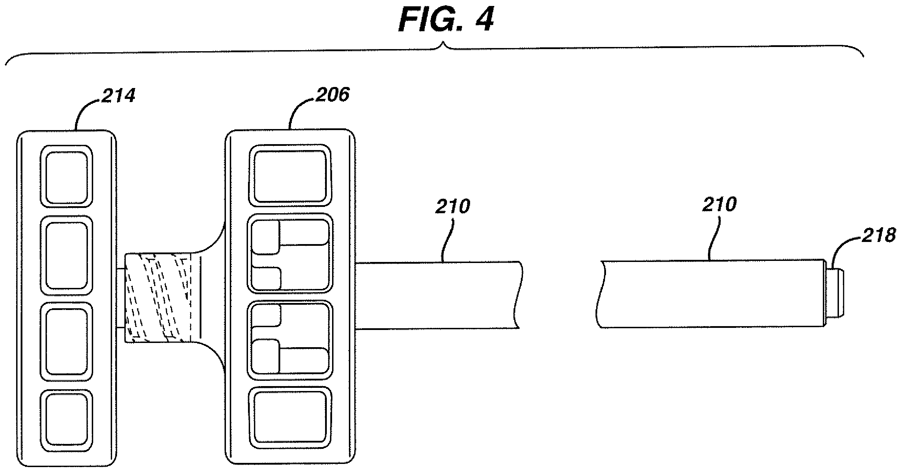

Referring now to Figs. 2-4, which illustrate a preferred embodiment of the present invention, the applicator 200 includes an outer delivery device 202 and an inner plunger device 204. The outer delivery device 202 has a handle element 206 at a proximal end 208 thereof, and an elongate tubular element 210 extending outwardly from the handle element to a distal end 212. Similarly, the inner plunger device 204 has a handle element 214 at a proximal end 216 thereof, and an elongate plunger element 218 extending outwardly from the handle element to a distal end 220.

The outer delivery device includes a channel 222 extending therethrough between the proximal 208 and distal 212 ends, with the size of the channel being defined by an inner diameter d₁ of the outer delivery device. Further, the device and channel therethrough include a proximal end region 250, a middle region 251 and a distal end region 252. Although the term "diameter" is used, it is to be understood that the term is not meant to be limited to circular cross-sections, although circular is the preferred embodiment. Further, the term "diameter" is not
intended to require a constant diameter along the length of the device, as will be described further below.

The outer diameter d₂ and length l₁ of the elongate tubular member 210 must be suitable for insertion through an endoscopic port such as a cannula or trocar. According to a preferred embodiment, the inner diameter d1 is approximately 3.8 mm, the outer diameter d₂ is approximately 4.8 mm, and the length l₁ is approximately 34 cm. With regard to handle element 206, any suitable configuration may be used that adequately allows the user to grasp and manipulate the outer delivery device. In the illustrated embodiment, the handle has a substantially disc-like portion 224 having a length l₂ of approximately 10 mm, and a diameter d₃ of approximately 2.5 cm, and an extension portion 226 at its proximal end.

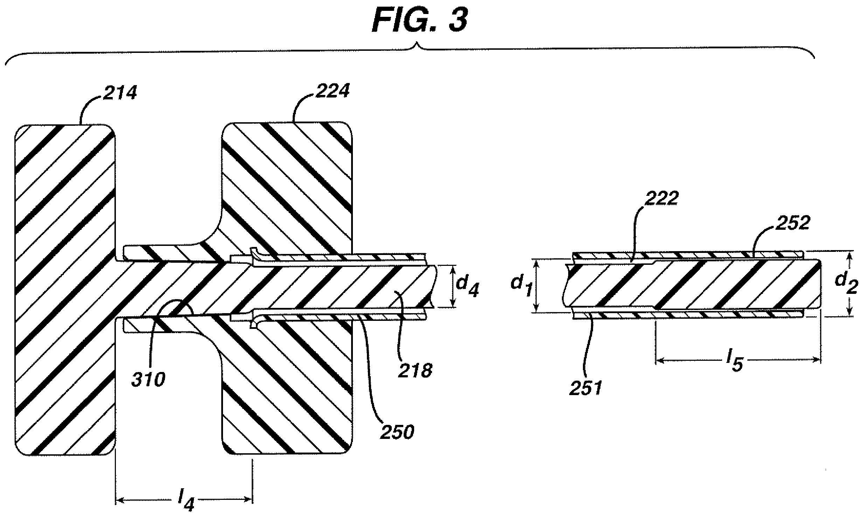

The elongate plunger element 218 of the inner plunger device 204 is dimensioned relative to the outer delivery device channel so as to be receivable therein, and slidable therein along a substantial portion of its length. In the illustrated embodiment, the length l₃ is further sized such that, when the elongate plunger element is inserted into the outer delivery channel, the distal end 220 of the elongate plunger element is at least substantially aligned with the distal end 212 of the outer delivery device, and preferably extends slightly beyond, as illustrated in greater detail in Fig. 3. The elongate plunger element is also defined by an outer diameter along its length l₃ and includes a proximal end region 230, a middle region 256 and a distal end region 257. In the illustrated embodiment, the outer diameter d₄ along most of the length is approximately 3.2 mm, whereas the outer diameter expands to approximately 3.7 mm along a distal portion of the length l₅. This allows the distal end of the elongate plunger element to have a closer tolerance relative to the channel of the outer delivery device, to enable fluid within the channel to more precisely be expressed with little or no leakage between the two elements and without significantly affecting the force required to slide the inner plunger element within the channel.

Referring now to the proximal end portion 230 of the elongate plunger element, in the illustrated embodiment shown in greater detail in Fig. 3, the diameter d₄ also expands outwardly in a tapered fashion, such as a luer taper, towards the proximal end. In a preferred embodiment, the expansion takes place along a length l₄ of approximately 10 mm, and to a maximum diameter of approximately 4.3 mm. This tapered proximal end portion 230 forms an interference fit with the outer delivery device, thereby preventing further movement relative to one another absent exertion of force by a user to separate the two. In this manner, it is ensured that the devices will not accidentally separate from one another when removed from the packaging as described above.

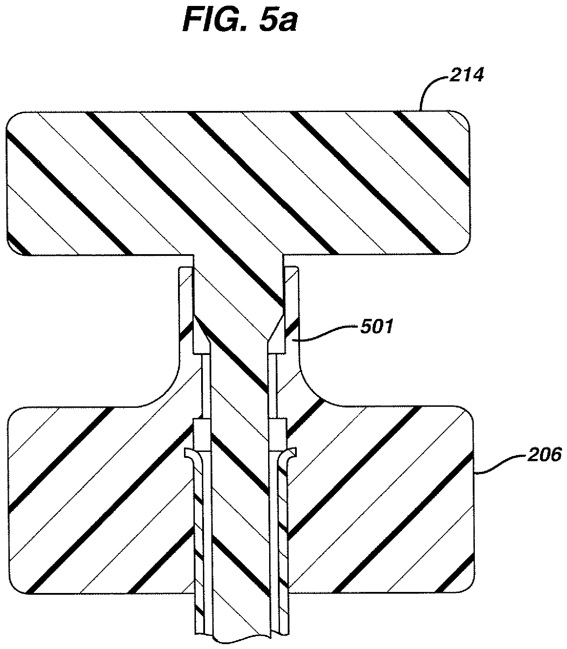

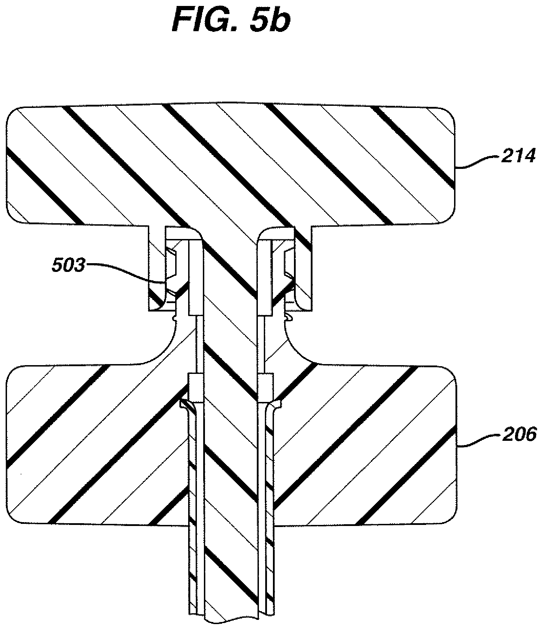

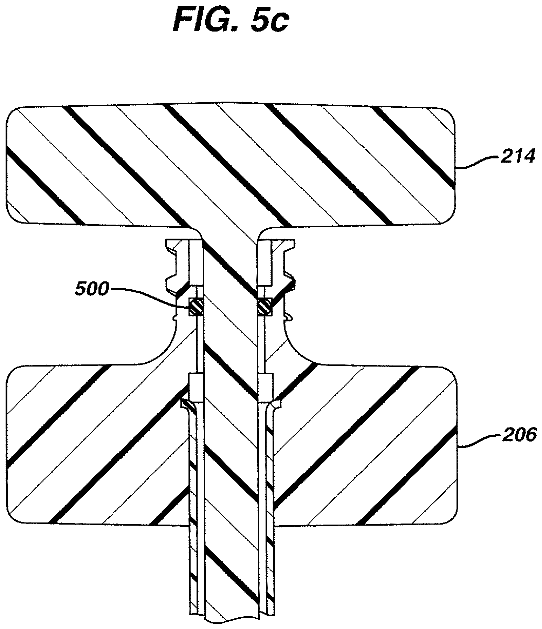

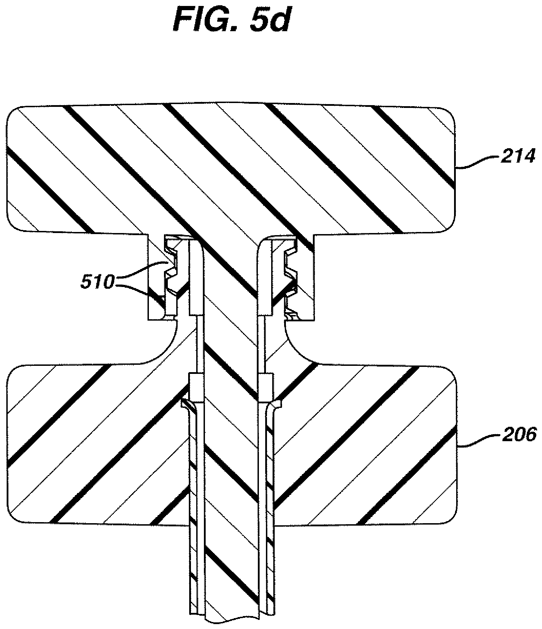

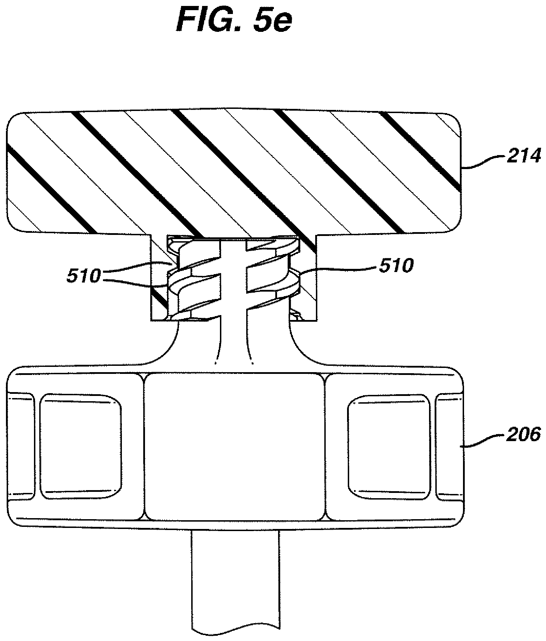

Although the illustrated embodiment shows an outwardly tapered or flared proximal end portion of the elongate plunger element, it is to be understood that any suitable means for forming an interference or locking fit as between the proximal end portions of the outer delivery device and inner plunger device can be used. For example, the respective handle portions could be appropriately configured so as to form an interference fit therebetween as shown in Figs. 5a (at reference numeral 501) and 5b (at reference numeral 503). Other means could include additional interference elements 500, such as gaskets or the like, that assist in forming an interference fit between the handle portions (i.e., Fig. 5c, or as between the outer delivery device and inner plunger device. Other means could also include complementary engaging threads 510 as shown in Figs 5d and 5e.

## Claims

1. A packaged endoscopic fluid applicator (200) comprising:
an outer delivery device (202) including a handle (206) at a proximal end (208) and an elongate tubular element (210) extending outwardly from the handle (206) to a distal end (212), wherein the outer delivery device (202) has a channel (222) extending therethrough between the proximal (208) and distal (212) ends;
an inner plunger device (204) including a handle element (214) at a proximal end (216) and an elongate plunger element (218) extending outwardly from the handle (214) to a distal end (220), wherein the elongate plunger element (218) is sized and shaped so as to be slidably receivable within the outer delivery device channel (222);
means at a proximal end region of the applicator (200) for forming an interference fit between the outer delivery device (202) and inner plunger device (204) substantially only when the elongate plunger element (218) is fully inserted within the outer delivery device channel (222); and
packaging for the endoscopic fluid applicator;
**characterized in that** the inner plunger device (204) is entirely removable from the outer delivery device (202) and the outer delivery device (202) and the inner plunger device (204) are pre-assembled with said interference fit within the package, the interference fit being sufficient to substantially prevent further movement of the inner plunger device (204) relative to the outer delivery device (202) absent exertion of a predetermined amount of force by a user.

2. The packaged applicator according to claim 1, wherein the means for forming an interference fit is an outwardly tapered proximal end region (230) of the elongate plunger element (218).

3. The packaged applicator according to claim 2, wherein the tapered end region (230) is a luer taper.

4. The packaged applicator according to claim 1, wherein the means for forming an interference fit further comprises at least one interference element positioned between the proximal end regions (208/216) of the outer delivery device channel (222) and the elongate plunger element (218).

5. The packaged applicator according to claim 1, wherein the means for forming an interference fit comprises a recess within the handle (214) of the inner plunger device (204) for receiving a corresponding extension portion of the handle (206) of the outer delivery device (202).

6. The packaged applicator according to claim 1, wherein the outer diameter of the elongate tubular element (210) is less than 5 mm.

7. The packaged applicator according to claim 5, wherein the inner diameter of the outer delivery device channel (222) is approximately 3.8 mm.

8. The packaged applicator according to claim 1, wherein the outer delivery device channel (222) is defined along its length by an inner diameter, the elongate plunger element (218) is defined along its length by an outer diameter, and the elongate plunger element (218) has a length such that, when received within the outer delivery device channel (222), it extends at least substantially to the distal end (212) of the outer delivery device (202).

9. The packaged applicator according to claim 8, wherein the elongate plunger element (218) has a proximal end region (230), a middle region (256), and a distal end region (257), and wherein the outer diameter is greater in the proximal end region (230) than in the middle region (256).

10. The packaged applicator according to claim 9, wherein the inner diameter of the outer delivery device channel (222) is substantially uniform along its length.

11. The packaged applicator according to claim 9, wherein the outer diameter of the elongate plunger element (218) at the middle region (256) is approximately 3.2 mm, and the outer diameter at the proximal end region (230) reaches approximately 4.3 mm.

12. The packaged applicator according to claim 11, wherein the outer diameter of the distal end region (257) of the elongate plunger element (218) is approximately 3.7 mm.

13. The packaged applicator according to claim 8, further comprising at least one interference element forming said interference fit as between the outer delivery device (202) and the inner plunger device (204) when the elongate plunger element (218) is substantially fully received within the outer delivery device channel (222).

14. The packaged applicator according to claim 8, wherein an outer diameter of the elongate tubular element (210) is less than 5 mm.

15. The packaged applicator according to claim 1, wherein the means for forming an interference fit between the outer delivery device (202) and the inner plunger device (204) is a threaded fit.

## Patentansprüche

1. Verpackter endoskopischer Fluid-Applikator (200), umfassend:
eine äußere Abgabevorrichtung (202) mit einem Griff (206) an einem proximalen Ende (208) und einem langgestreckten röhrenförmigen Element (210), das sich vom Griff (206) nach außen zu einem distalen Ende (212) erstreckt, wobei die äußere Abgabevorrichtung (202) einen Kanal (222) aufweist, der sich zwischen dem proximalen Ende (208) und dem distalen Ende (212) dort hindurch erstreckt;
eine innere Kolbenvorrichtung (204) mit einem Griffelement (214) an einem proximalen Ende (216) und einem langgestreckten Kolbenelement (218), das sich vom Griff (214) nach außen zu einem distalen Ende (220) erstreckt, wobei das langgestreckte Kolbenelement (218) eine solche Größe und Form aufweist, dass es verschiebbar im Kanal (222) der äußeren Abgabevorrichtung aufgenommen werden kann;
Mittel an einer proximalen Endregion des Applikators (200) zur Bildung eines Presssitzes zwischen der äußeren Abgabevorrichtung (202) und der inneren Kolbenvorrichtung (204), im Wesentlichen nur dann, wenn das langgestreckte Kolbenelement (218) vollständig in den Kanal (222) der äußeren Abgabevorrichtung eingeführt ist; und
eine Verpackung für den endoskopischen Fluid-Applikator;
**dadurch gekennzeichnet, dass** die innere Kolbenvorrichtung (204) vollständig aus der äußeren Abgabevorrichtung (202) entfernbar ist und sich die äußere Abgabevorrichtung (202) und die innere Kolbenvorrichtung (204) mit dem Presssitz vormontiert in der Verpackung befinden, wobei der Presssitz ausreicht, um eine weitere Bewegung der inneren Kolbenvorrichtung (204) bezüglich der äußeren Abgabevorrichtung (202) in Abwesenheit einer vorbestimmten Kraftmenge, die von einem Benutzer ausgeübt wird, im Wesentlichen zu verhindern.

2. Verpackter Applikator nach Anspruch 1, wobei das Mittel zur Bildung eines Presssitzes eine nach außen verjüngte proximale Endregion (230) des langgestreckten Kolbenelements (218) ist.

3. Verpackter Applikator nach Anspruch 2, wobei die verjüngte Endregion (230) ein Luer-Kegel ist.

4. Verpackter Applikator nach Anspruch 1, wobei das Mittel zur Bildung eines Presssitzes ferner mindestens ein Zwischenelement umfasst, das zwischen den proximalen Endregionen (208/216) des Kanals (222) der äußeren Abgabevorrichtung und dem langgestreckten Kolbenelement (218) angeordnet ist.

5. Verpackter Applikator nach Anspruch 1, wobei das Mittel zur Bildung eines Presssitzes eine Aussparung im Griff (214) der inneren Kolbenvorrichtung (204) zur Aufnahme eines entsprechenden Verlängerungsabschnitts des Griffs (206) der äußeren Abgabevorrichtung (202) umfasst.

6. Verpackter Applikator nach Anspruch 1, wobei der Außendurchmesser des langgestreckten röhrenförmigen Elements (210) weniger als 5 mm beträgt.

7. Verpackter Applikator nach Anspruch 5, wobei der Innendurchmesser des Kanals (222) der äußeren Abgabevorrichtung ungefähr 3,8 mm beträgt.

8. Verpackter Applikator nach Anspruch 1, wobei der Kanal (222) der äußeren Abgabevorrichtung entlang seiner Länge von einem Innendurchmesser definiert wird, das langgestreckte Kolbenelement (218) entlang seiner Länge von einem Außendurchmesser definiert wird und das langgestreckte Kolbenelement (218) eine solche Länge aufweist, dass es sich, wenn es im Kanal (222) der äußeren Abgabevorrichtung aufgenommen ist, mindestens im Wesentlichen zum distalen Ende (212) der äußeren Abgabevorrichtung (202) erstreckt.

9. Verpackter Applikator nach Anspruch 8, wobei das langgestreckte Kolbenelement (218) eine proximale Endregion (230), eine mittlere Region (256) und eine distale Endregion (257) aufweist, und wobei der Außendurchmesser in der proximalen Endregion (230) größer als in der mittleren Region (256) ist.

10. Verpackter Applikator nach Anspruch 9, wobei der Innendurchmesser des Kanals (222) der äußeren Abgabevorrichtung entlang seiner Länge im Wesentlichen gleichförmig ist.

11. Verpackter Applikator nach Anspruch 9, wobei der Außendurchmesser des langgestreckten Kolbenelements (218) an der mittleren Region (256) ungefähr 3,2 mm beträgt und der Außendurchmesser an der proximalen Endregion (230) ungefähr 4,3 mm erreicht.

12. Verpackter Applikator nach Anspruch 11, wobei der Außendurchmesser der distalen Endregion (257) des langgestreckten Kolbenelements (218) ungefähr 3,7 mm beträgt.

13. Verpackter Applikator nach Anspruch 8, ferner umfassend mindestens ein Zwischenelement, das den Presssitz wie zwischen der äußeren Abgabevorrichtung (202) und der inneren Kolbenvorrichtung (204) bildet, wenn das langgestreckte Kolbenelement (218) im Wesentlichen vollständig im Kanal (222) der äußeren Abgabevorrichtung aufgenommen ist.

14. Verpackter Applikator nach Anspruch 8, wobei ein Außendurchmesser des langgestreckten röhrenförmigen Elements (210) kleiner als 5 mm ist.

15. Verpackter Applikator nach Anspruch 1, wobei das Mittel zur Bildung eines Presssitzes zwischen der äußeren Abgabevorrichtung (202) und der inneren Kolbenvorrichtung (204) eine Gewindepassung ist.

## Revendications

1. Applicateur de fluide endoscopique emballé (200) comprenant :
un dispositif de distribution extérieur (202) comportant une poignée (206) à une extrémité proximale (208) et un élément tubulaire allongé (210) s'étendant vers l'extérieur depuis la poignée (206) jusqu'à une extrémité distale (212), le dispositif de distribution extérieur (202) présentant un canal (222) s'étendant à travers lui entre les extrémités proximale (208) et distale (212) ;
un dispositif de plongeur intérieur (204) comportant un élément de poignée (214) à une extrémité proximale (216) et un élément de plongeur allongé (218) s'étendant vers l'extérieur depuis la poignée (214) jusqu'à une extrémité distale (220), l'élément de plongeur allongé (218) étant dimensionné et formé de manière à pouvoir être reçu par coulissement à l'intérieur du canal (222) du dispositif de distribution extérieur ;
un moyen au niveau d'une région d'extrémité proximale de l'applicateur (200) pour former un ajustement serré entre le dispositif de distribution extérieur (202) et un dispositif de plongeur intérieur (204) substantiellement uniquement lorsque l'élément de plongeur allongé (218) est complètement inséré à l'intérieur du canal (222) du dispositif de distribution extérieur ; et
un emballage pour l'applicateur de fluide endoscopique ;
**caractérisé en ce que** le dispositif de plongeur intérieur (204) peut être entièrement retiré du dispositif de distribution extérieur (202) et le dispositif de distribution extérieur (202) et le dispositif de plongeur intérieur (204) sont préassemblés avec ledit ajustement serré à l'intérieur de l'emballage, l'ajustement serré étant suffisant pour empêcher sensiblement tout mouvement supplémentaire du dispositif de plongeur intérieur (204) par rapport au dispositif de distribution extérieur (202) en l'absence de l'application d'une quantité de force prédéterminée par un utilisateur.

2. Applicateur emballé selon la revendication 1, dans lequel le moyen de formation d'un ajustement serré est une région d'extrémité proximale effilée vers l'extérieur (230) de l'élément de plongeur allongé (218) .

3. Applicateur emballé selon la revendication 2, dans lequel la région d'extrémité effilée (230) est un cône de Luer.

4. Applicateur emballé selon la revendication 1, dans lequel le moyen de formation d'un ajustement serré comprend en outre au moins un élément d'ajustement positionné entre les régions d'extrémité proximale (208/216) du canal (222) du dispositif de distribution extérieur et l'élément de plongeur allongé (218).

5. Applicateur emballé selon la revendication 1, dans lequel le moyen de formation d'un ajustement serré comprend un renfoncement à l'intérieur de la poignée (214) du dispositif de plongeur intérieur (204) pour recevoir une partie d'extension correspondante de la poignée (206) du dispositif de distribution extérieur (202).

6. Applicateur emballé selon la revendication 1, dans lequel le diamètre extérieur de l'élément tubulaire allongé (210) est inférieur à 5 mm.

7. Applicateur emballé selon la revendication 5, dans lequel le diamètre intérieur du canal (222) du dispositif de distribution extérieur mesure environ 3,8 mm.

8. Applicateur emballé selon la revendication 1, dans lequel le canal (222) du dispositif de distribution extérieur est défini le long de sa longueur par un diamètre intérieur, l'élément de plongeur allongé (218) est défini le long de sa longueur par un diamètre extérieur, et l'élément de plongeur allongé (218) présente une longueur telle que, lorsqu'il est reçu à l'intérieur du canal (222) du dispositif de distribution extérieur, il s'étende au moins sensiblement jusqu'à l'extrémité distale (212) du dispositif de distribution extérieur (202).

9. Applicateur emballé selon la revendication 8, dans lequel l'élément de plongeur allongé (218) présente une région d'extrémité proximale (230), une région centrale (256), et une région d'extrémité distale (257), et dans lequel le diamètre extérieur dans la région d'extrémité proximale (230) est supérieur au diamètre dans la région centrale (256).

10. Applicateur emballé selon la revendication 9, dans lequel le diamètre intérieur du canal (222) du dispositif de distribution extérieur est sensiblement uniforme le long de sa longueur.

11. Applicateur emballé selon la revendication 9, dans lequel le diamètre extérieur de l'élément de plongeur allongé (218) au niveau de la région centrale (256) mesure environ 3,2 mm, et le diamètre extérieur au niveau de la région d'extrémité proximale (230) atteint approximativement 4,3 mm.

12. Applicateur emballé selon la revendication 11, dans lequel le diamètre extérieur de la région d'extrémité distale (257) de l'élément de plongeur allongé (218) mesure approximativement 3,7 mm.

13. Applicateur emballé selon la revendication 8, comprenant en outre au moins un élément d'ajustement formant ledit ajustement serré entre le dispositif de distribution extérieur (202) et le dispositif de plongeur intérieur (204) lorsque l'élément de plongeur allongé (218) est sensiblement reçu entièrement l'intérieur du canal (222) du dispositif de distribution extérieur.

14. Applicateur emballé selon la revendication 8, dans lequel un diamètre extérieur de l'élément tubulaire allongé (210) est inférieur à 5 mm.

15. Applicateur emballé selon la revendication 1, dans lequel le moyen pour former un ajustement serré entre le dispositif de distribution extérieur (202) et le dispositif de plongeur intérieur (204) est un ajustement fileté.
